# EUROPÄISCHE PATENTANMELDUNG

(11) **EP 3 020 680 A1**
(43) Veröffentlichungstag der Anmeldung: **18.05.2016**
(21) Anmeldenummer: 15194187.9
(22) Anmeldetag: 12.11.2015
(51) Int. Cl.: B67C 3/26, B67C 3/30, B67D 1/08

(54) **STERIL-BEFÜLL- BZW. SPÜLEINRICHTUNG**

(30) Priorität: 13.11.2014 AT 508282014
(71) Anmelder: Grünewald Fruchsaft GmbH, 8510 Stainz (AT)
(72) Erfinder: Schröttner, Stefan, 8501 Lieboch (AT)
(74) Vertreter: Wildhack & Jellinek

(57) **Zusammenfassung**

Die Erfindung betrifft eine Sterilbefüll- und -spüleinrichtung für kontaminationsfreies Befüllen von mit sensiblen fließfähigen Produkten zu beschickenden Behältern od. dgl. mit einem fluiden, sterilen Medium, welche einen Spül- und Füllkopf (5) mit in seinen Innenraum (50) mündenden Stutzen (6, 7, 10) mit Sperrventilen (11, 12, 13) aufweist, wobei zwei Stutzen (6, 7) für Zu- und Abfuhr von Heißdampf (60) und einer (10) für den Durchlauf des Fluidmediums (100) vorgesehen sind, und weiters mit einem Anschlussadapterstück (2) sowie mit einer Betätigungseinrichtung (8) für die Bewegung eines Betätigungsstiftes (9) für das Einlassventil des Behälters (3), wobei nach Zusammenschluss zu einem Ensemble (4) von Spül- und Füllkopf (5) mit dem Einlassventil (1) der Innenraum (50) des Spül- und Füllkopfes (5) und das Einlassventil (1) des Behälters (3) und nach Niederfahren des Ventilbetätigungsstiftes (9) der Einlassventilinnenraum (33) zeitablaufgesteuert, mit Heißdampf (60) bis zur gesicherten Sterilisierung des Inneren dieses Ensembles (4) beschickbar ist, wonach die Befüllung des Behälters (3) mit dem fluiden Medium (100) vornehmbar ist. Sie betrifft weiters den Spül- und Füllkopf (5) selbst und die Verwendung der neuen Einrichtung.

## Beschreibung

Aus dem Stand der Technik ist es bekannt, dass Anschlüsse zum sterilen Befüllen und Entleeren von Gebinden und Behältern, aber auch beim Koppeln von Leitungen in vielen Bereichen der Lebensmittelindustrie und der Pharmaindustrie desinfiziert werden müssen, bevor fluide Medien, wie Gase, aber auch Flüssigkeiten usw. durch Leitungen sicher befördert werden dürfen, um dadurch das Risiko einer mikrobiologischen Kontamination zu minimieren bzw. auszuschalten.

Das gilt grundlegend für alle Anschlüsse und Ventile, bei welchen es notwendig ist, diese im laufenden Betrieb an- und abzukoppeln. Für diese Desinfektion der Anschlüsse und Ventile gibt es einerseits diverse Desinfektionslösungen, wie z.B. Ethanol. Aber gerade bei mechanisch komplex aufgebauten, selbstschließenden Anschlüssen und Ventilen ist es fast unmöglich, mit der Alkohol-Lösung sämtliche Flächen und Teile der Anschlüsse bzw. der Ventile zu erreichen, welche dann beim Befüllvorgang mit dem jeweiligen Medium in Berührung kommen. Fertigungsbereiche, in welchen solche Anschlüsse entkoppelt werden, entsprechen in den seltensten Fällen einem Reinraum. So kann es durch ein unsteriles Koppeln von Ventilen beim Fördern der Medien schnell zu einer Kontamination der Medien kommen, was in weiterer Folge die Produktsicherheit gefährdet.

Weiters ist es bekannt, dass es spezielle Sterilfilter gibt, die eine solche Kontamination verhindern. Diese sind aber laufend, z.B. bei jedem Containerreinigungsvorgang, zu wechseln. Das stellt sich wiederum als zeit- und kostenintensiv dar. Auch ein schnelles Verstopfen des Filters in Folge des Eindringens von Flüssigkeiten ist leicht möglich.

Weiters besteht die Möglichkeit, eine Desinfektion mittels überhitztem bzw. Heiß-Dampf vorzunehmen. Auch hier gibt es jedoch das Problem, dass selbstschließende Anschlüsse nicht an allen Stellen mit dem Heißdampf erreicht werden können. Vor allem aber ist dies in einem produktiven Betrieb praktisch kaum zu überwachen bzw. zu überprüfen.

Der Zeitraum und die Stellen die notwendig sind, die Anschlüsse und Ventile mit dem Heißdampf zu beaufschlagen, können zwar dem Bediener vorgegeben werden, dies lässt sich im bzw. für den laufenden Betrieb jedoch nicht kontrollieren.

Weiters wäre der Zeitraum, in welchem ein Verschluss bzw. ein Ventil mit Heißdampf beaufschlagt werden müsste, viel zu lange, wenn man auch jene Teile auf die nötige hohe Temperatur bringen müsste, welche nicht direkt vom Heißdampf erreicht werden, weil die Anschlussstücke im nicht gekoppelten Zustand verschlossen sind.

Ziel der Erfindung ist es, den Sterilisationsprozess nach erfolgtem Ankoppeln eines Befüllungskopfes an den Anschluss bzw. an das Ventil des Anschlusses eines Containers mit hoher Sicherheit zu verwirklichen, und zwar mittels Heißdampf, noch bevor es zum Durchfluss der jeweils in den Behälter od. dgl. zu fördernden Medien kommt, welche insbesondere sterile Gase, wie z.B. sterile Luft, Stickstoff oder CO₂ sein können, jedoch auch beispielsweise klare Nahrungsmittellösungen, Säfte, Extraktlösungen od. dgl.

Gegenstand der vorliegenden Erfindung ist eine neue Steril-Befüll- bzw. Spüleinrichtung für ein kontaminationsfreies Befüllen bzw. Spülen von mit sensiblen fließfähigen Produkten, insbesondere des Lebens- und Arzneimittelsektors, zu beschickenden Behältern, Tanks, Containern, Rohren oder Rohrsystemen mit einem fluiden Medium aus der Gruppe Flüssigkeiten und Gase, insbesondere mit einem Inert- bzw. Sterilgas, wie Stickstoff oder Kohlendioxid,
umfassend einen Spül- und Füllkopf für die Führung bzw. Zuführung des fluiden Mediums und einem mit dem Spül- und Füllkopf fluiddicht zu einem Ensemble verbindbaren Anschlussstutzen des Behälters od.dgl. mit Einlassventil mit nach außen hin druckfederbelastetem Ventilkörper, welche **dadurch gekennzeichnet** ist,
- dass der, vorzugsweise im Wesentlichen zylindrische, Spül- und Füllkopf zumindest drei dort seitlich einmündende, mit seinem Innenraum in Verbindung stehende, selbst jeweils ein Sperrventil aufweisende, außen an flexible Rohre oder Schläuche angeschlossene, Stutzen aufweist, von welchen zwei jeweils für Zufuhr- und Abfuhr von überhitztem bzw. Heiß-Dampf und der dritte für den Ein- und Durchlauf des jeweiligen Fluidmediums vorgesehen, sind, wobei gegebenenfalls der Dampfabführ-Stutzen einen geringeren Querschnitt aufweist als der Dampfzufuhr-Stutzen,
- dass er weiters an seinem offenen unteren Ende mit einem Anschlussadapterstück ausgebildet ist und an seinem dem offenen Ende gegenüberliegenden Ende eine, gegebenenfalls mit einer Messeinheit, beispielsweise Manometer und/oder Thermometer, für die Ermittlung von Druck und/oder Temperatur, beispielsweise im Spül- und FüllkopfInnenraum ausgestattete, Betätigungseinrichtung für das Hoch- oder Niederfahren eines für das Betätigen, insbesondere Öffnen, des druckfederbelasteten Ventilkörpers des Einlassventils am Anschlussstutzen des Behälters od. dgl. vorgesehenen, mittig, insbesondere achsial, in den Innenraum des Spül- und Füllkopfes ragenden Einlassventil-Betätigungsstiftes bzw. -stabes aufweist, und
- dass erst nach erfolgtem, vorzugsweise übergreifendem, Zusammenschluss zum Ensemble von Spül- und Füllkopf mit seinem Anschluss-Adapterstück mit dem Einlassventil am Anschlussstutzen des Behälters od. dgl. über dessen Gegenadapter der gesamte Innenraum des Spül- und Füllkopfes und der Außenraum um den Gegenadapter sowie das Einlassventil des Anschlussstutzens des Behälters od. dgl. und nach Niederfahren des Einlassventil-Betätigungsstiftes auch der Innenraum des Einlassventils mit dem Einlassventilkörper zeitvorgegeben, insbesondere zeitablaufgesteuert, mit dem Heißdampf bis zur voll gesicherten Sterilisierung des Inneren dieses Ensembles beschickbar ist,
- wonach erst die Befüllung und Spülung des Behälters od. dgl. mit dem jeweils vorgesehenen fluiden Medium vornehmbar ist.

Das oben erwähnte Druck- und/oder Temperaturüberwachungsgerät im System, also z.B. im Kopfbehälter, Rohr od. dgl., ist aber keinesfalls zwingend vorgesehen, es kann aber z.B. auch in der sterilen Leitung zum Ventil angeordnet sein.

An das Anschlussstück bzw. an den Anschlussstutzen eines Behälters, Containers od. dgl. bzw. an dessen Einlassventil wird ein Gegenstück mit der Zuleitung des Mediums, nämlich der Spül- und Füllkopf der neuen Einrichtung angebracht. Am Spül- und Füllkopf ist ein Adapterstück angeordnet, wodurch ermöglicht ist, neben dem eigentlichen fluiden Medium, welches gefördert werden soll, Heißdampf sowohl zu dem Anschlussstutzen als auch zu dem und in den Spül- und Füllkopf zu befördern, beziehungsweise diese beiden Komponenten der neuen Einrichtung mit dem Heißdampf zu beaufschlagen. Nach dem Anschluss des Spül- und Füllkopfes und dessen mit ihm integrierten Adapters an den Anschlussstutzen des Behälters, Containers od. dgl. werden sowohl der Anschlussstutzen mit dem Einlassventil als auch der Spül- und Füllkopf mit dem Heißdampf beaufschlagt.

Erst nach Beendigung des Sterilisationsvorganges, selbstverständlich auch des Innenraums des Adapterstücks, das auf dem Spül- und Füllkopf befestigt ist und diesen vollkommen umschließt, drückt der Einlassventil-Betätigungsstift nach unten, öffnet dabei das Einlassventil des Anschlussstutzens des Behälters od. dgl. und ermöglicht so ein kurzes Einströmen bzw. den Durchfluss des Heißdampfes. Erst wenn dieser Innenraum steril ist, wird letztlich dann die Verbindung zur Förderung des eigentlichen fluiden Mediums hergestellt.

Dieser Ablauf lässt sich steuertechnisch automatisiert regeln, auf Dauer gewährleisten und, je nach Ausführung der Steuerung, auch protokollieren. Wird dieser Prozess rein mechanisch, also ohne Steuerungsautomatik umgesetzt, so wird jedenfalls zumindest gewährleistet, dass jeweils der Anschlussstutzen des Behälters od. dgl. und der Spül- und Füllkopf, die letztlich mit dem fluiden Medium in Berührung kommen, vor Öffnung des Einlassventils des Anschlussstutzens und somit an allen erforderlichen sensiblen Stellen mittels des Heißdampfes voll sterilisiert werden.

Erfolgt eine steuerungsunterstützte Sterilisierung mittels Heißdampf nach dem beschriebenen Prinzip, so hat der Bediener lediglich den Spül- und Füllkopf mit integriertem Adapterstück auf den Anschlussstutzen des Behälters od. dgl. bzw. auf dessen Gegenadapter zu stecken. Dieser Arbeitsgang ist an sich immer notwendig.

Danach kann der Reinigungs- bzw. Sterilisierungs- und Befüllprozess starten. Die Steuerung beginnt damit, den Heißdampf über das Adapterstück des Spül- und Füllkopfes zu dem Anschlussstutzen des Behälters od. dgl. zu führen. Dies erfolgt jeweils mit vorgegebenen Zeiten und bei jeweils vorgesehenen Temperaturen.

Zeitverzögert nach erfolgtem Sterilisationsvorgang, die hierfür nötige Zeitdauer wird jeweils nach entsprechender Validierung festgesetzt, öffnet die Mechanik die Verbindung zwischen dem Spül- und Füllkopf- und dem Anschlussstutzen des Behälters, Containers od. dgl. und somit den Durchfluss für das jeweils vorgesehene eigentliche fluide Medium, sodass es gefördert werden kann.

Der gesamte Ablauf kann, wie beschrieben, ohne Automatik, also rein mechanisch, im Teilautomatik- oder Vollautomatikbetrieb bis hin zu Dokumentation und elektronischer Überwachung erfolgen und geregelt werden.

Gemäß einer bevorzugten Ausführungsform kann im Rahmen der Erfindung zur Steuerung vorgesehen sein, dass die neue Steril-Befüll- und Spüleinrichtung eine zentrale Steuerungseinheit aufweist, welche über (Funk-)Datenleitungen mit den zeitvorgegeben, insbesondere zeitablaufgesteuert, aufeinander abgestimmt betätigbaren Ventilen für die Heißdampf-Führung und mit dem Ventil für den Ein- und Durchlauf des fluiden Mediums sowie mit der Betätigungseinrichtung für den Einlassventil-Betätigungsstift bzw. -stab datentransfer-verbunden ist.

Des weiteren kann vorteilhafterweise dafür gesorgt sein, dass beispielsweise an zumindest einer, insbesondere durch Kondensation des Heißdampfes gefährdeten, sensiblen Stelle des Innenraums des Spül- und Füllkopfes ein Thermosensor angeordnet ist, der über eine Temperaturmesseinheit der Stift- bzw. Stabbetätigungseinrichtung mit der zentralen Steuerungseinheit datentransfer-verbunden ist.

Ein weiterer wesentlicher Gegenstand der Erfindung ist der Spül- und Füllkopf der neuen Steril-Befüll- und Spüleinrichtung, welcher dadurch gekennzeichnet ist, dass er im Wesentlichen Zylinderform mit zylindrischem Innenraum aufweist, in welchen drei an flexible Rohre oder Schläuche angeschlossene Stutzen seitlich einmünden, von welchen zwei jeweils für Zufuhr- und Abfuhr von überhitztem bzw. Heiß-Dampf und der dritte für den Ein- und Durchlauf des jeweiligen Fluidmediums vorgesehen, sind,
- dass er weiters an seinem offenen Ende mit einem Anschlussadapterstück ausgebildet ist und an seinem dem offenen Ende gegenüberliegenden Ende eine, gegebenenfalls mit Druck- und/oder Temperatur-Messeinheit ausgestattete, Betätigungseinrichtung für das Hoch- oder Niederfahren des für das Betätigen, insbesondere Öffnen, des druckfederbelasteten Ventilkörpers des Einlassventils am bzw. im Anschlussstutzen des Behälters od. dgl. vorgesehenen, mittig, insbesondere achsial, in den Innenraum des Spül- und Füllkopfes ragenden Einlassventil-Betätigungsstiftes bzw. -stabes aufweist, und
- dass er mit seinem Anschlussadapterstück an den Anschlussstutzen mit dem Einlassventil des zu befüllenden Behälters, Containers od. dgl. fluiddicht anschließbar ist.

Der Anschlussstutzen besitzt ein handelsübliches Ventil. Ebenso ist der Gegenadapter, des Spül- und Füllkopfes welcher selbstarretierend über das Einlassventil geschoben werden kann, ein handelsübliches Objekt.

Einen weiteren Gegenstand der vorliegenden Erfindung bildet ein Verfahren zum Steril-Befüllen und -Spülen von mit sensiblen fließfähigen Produkten, insbesondere des Lebens- und Arzneimittelsektors, beschickbaren Behältern, Tanks, Containern, Rohren oder Rohrsystemen mit einem fluiden Medium aus der Gruppe Flüssigkeiten und Gase, insbesondere mit einem Inertgas, wie Stickstoff oder Kohlendioxid. Dieses ist dadurch gekennzeichnet,
- dass der Spül- und Füllkopf der wie bisher beschriebenen Steril-Befüll- und Spüleinrichtung fluiddicht an den Anschlussstutzen des Behälters od. dgl. bzw. an dessen Ventil mit druckfederbelastetem Ventilkörper angeschlossen wird,
- dass danach bei geschlossenem Ventil von dessen Mediumzufuhr-Stutzen und bei geöffnetem Ventil des Dampfzufuhr-Stutzens und bei zumindest zeitweise geöffnetem Ventil des Dampfabfuhr-Stutzens überhitzter bzw. Heiß-Dampf so lange in und durch den Innenraum des Spül- und Füllkopfes und auf die Außenflächen des Anschlussstutzens des Behälters, Containers od. dgl. von dessen geschlossenem Einlassventil und dessen druckfederbelastetem Ventilkörper spülend geleitet wird, bis dort volle Sterilität gewährleistet ist,
- dass dann nach Öffnung des Einlassventils des Anschlussstutzens des Behälters, Containers od. dgl. der Einlassventil-Innenraum mit dem Heißdampf bis zur Einreichung voller Sterilität dortselbst beaufschlagt wird, und
- dass schließlich nach Schließen der Ventile in den Stutzen des Spül- und Füllkopfes und nach Öffnen von dessen Zufuhrventil das fluide Medium über das mittels Ventilbetätigungsstift bzw. -stab des Spül- und Füllkopfes geöffnet gehaltene Einlassventil des Anschlussstutzens des Behälters, Containers od. dgl. in denselben einlaufen gelassen wird.

Schließlich ist als weiterer Gegenstand der Erfindung die Verwendung einer Steril-Befüll-bzw. Spüleinrichtung bzw. eines wie oben beschriebenen Spül- und Füllkopf für ein kontaminationsfreies Befüllen bzw. Spülen von mit sensiblen fließfähigen Produkten, insbesondere des Lebens- und Arzneimittelsektors, zu beschickenden Behältern, Tanks, Containern, Rohren oder Rohrsystemen, insbesondere unter Durchführung des wie oben beschriebenen Verfahrens anzuführen.

Die Erfindung wird anhand der Zeichnung näher erläutert:
Es zeigen die Fig. 1 die neue Steril-Befüll- und Spüleinrichtung vor dem Anschließen von deren Spül- und Füllkopf an den Anschlussstutzen des Behälters, Containers od. dgl., die Fig. 2 die neue Einrichtung im Zustand des erfolgten Zusammenschlusses des Spül- und Füllkopfes mit dem Anschlussstutzen des Behälters, Containers od. dgl. und vor der Beschickung dieses "Ensembles" mit dem übersättigten bzw. Heiß-Dampf für dessen Dekontamination bzw. Sterilisierung, die Fig. 3 dieses "Ensemble" zu Beginn und während des Einlassens des Heißdampfes in das Innere des an den Anschlussstutzen des Behälters, Containers od. dgl. und an dessen Einlassventil angeschlossenen Spül- und Füllkopfes, wobei der Heißdampf jedenfalls mit diesem noch geschlossen gehaltenen Einlassventil in intensive Berührung kommt, die Fig. 4 das genannte Ensemble, wobei das Einlassventil des Anschlussstutzens geöffnet ist und der Heißdampf das gesamte Einlassventil bzw. dessen Innenraum mit Ventilkörper und Ventilfeder sterilisierend umspült und die Fig. 5 das genannte Ensemble im Stadium nach erfolgter Schließung der Heißdampf-Zufuhr und -Spülung und bei Einströmen des jeweils vorgesehenen fluiden Mediums durch den Spül- und Füllkopf und den Behälter-Anschlussstutzen in den jeweiligen Behälter, Container od. dgl.

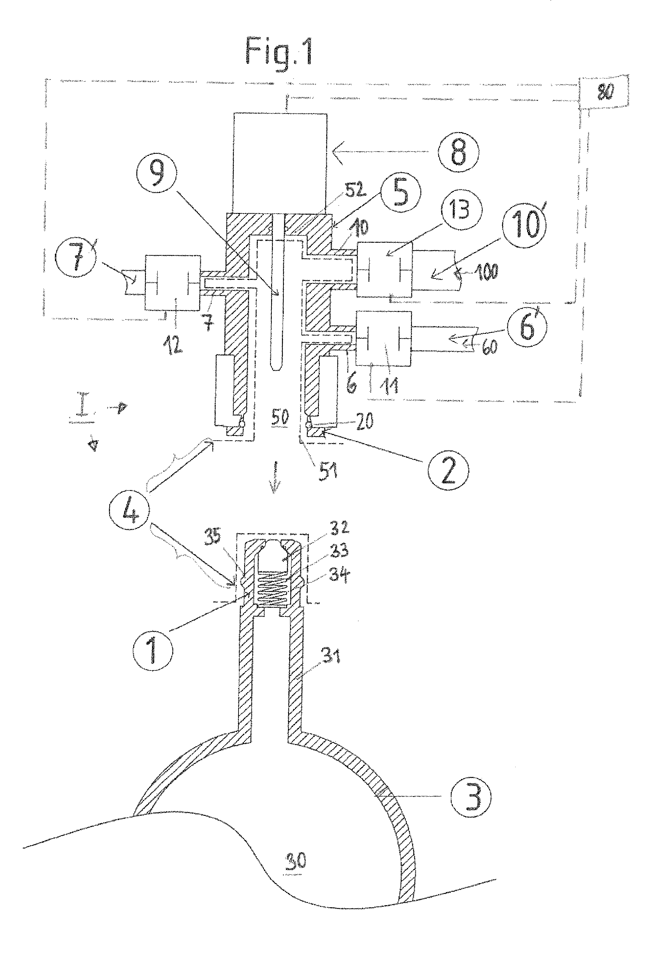

Die Fig. 1 zeigt die neue Befüll- und Spüleinrichtung I und zwar einerseits den neuen Spül- und Füllkopf 5 mit Innenraum 50 und anderseits den Behälter 3 mit Anschlussstutzen 31 und Einlassventil 1 mit in dessen Innenraum 34 angeordneter Druckfeder 33 und mittels derselben nach außen hin druckbelastetem Ventilkörper 32.

Der Anschlussstutzen 31 ist mit einem Gegenadapter 35 ausgestattet, welcher mit dem Adapterstück 2 mit Dichtring 20 am unteren Ende 51 des Spül- und Füllkopfes 5 dichtend zu kooperieren imstande ist.

In den Innenraum 50 des im Wesentlichen zylindrischen Spül- und Füllkopfes 5 münden jeweils von der Seite her mit, vorzugsweise flexiblen, Leitungsrohren 6' und 7' verbundene Stutzen 6 und 7 mit Sperrventilen 11 und 12, durch welche Stutzen der für die Sterilisation vorgesehene Heißdampf 60 zu- und abführbar ist. Hierbei kann es günstig sein, wenn der Abführ-Stutzen 7 einen geringeren Durchmesser aufweist, als der Zuführstutzen 6 für den Heißdampf 60.

Weiters mündet dort ein für die Zufuhr des fluiden Mediums 100, insbesondere Sterilgas, vorgesehener, an die Zuleitung 10' angeschlossener Stutzen 10, ebenfalls mit Sperrventil 13 ein.

Am dem dem offenen unteren Ende 51 des Spül- und Füllkopfes 5 gegenüberliegenden, oberen Ende 52 desselben ist eine Stabschiebe- und Einlassventil-Betätigungsseinrichtung 8 angeordnet, mittels welcher der Betätigungsstab bzw. -stift 9 für das Einlassventil 1 des Behälters 3 bzw. für dessen Ventilkörper 32 hoch- oder niederfahren gelassen werden kann.

Die Stab-Betätigungseinrichtung 8 ist über eine Datentransferleitung oder Funk - hier in unterbrochener Linie - mit der zentralen Steuerungseinrichtung 80 verbunden, die ihrerseits weiters mit den Sperrventilen 11, 12 und 13 des Spül- und Füllkopfes 5 und datentransfer-verbunden ist.

Die Software der Steuerungseinrichtung 80 sorgt für die, insbesondere zeitlich, aufeinander abgestimmten Bewegungen der Sperrventile 11, 12 und 13 sowie des Einlassventill-Betätigungsstabes 9 mittels der Einlassventill-Betätigungseinrichtung.

Im Innenraum 50 des Füll- uns Spülkopfes 5 kann gegebenenfalls ein Thermosensor für die Ermittlung und Einstellung der dort herrschenden Temperatur zumindest während der Heißdampf-Beschickungs-Phase angeordnet sein, der über die Stab9-Betätigungseinrichtung 8 ebenfalls mit der Steuerungseinrichtung 80 datentransfer-verbunden ist.

Das Bezugszeichen 4 deutet den für die Inbetriebnahme der neuen Einrichtung I in Folge vorzunehmenden Fluiddicht-Anschluss des Spül- und Füllkopfes 5 an den Anschlussstutzen 31 des Behälters 3 od. dgl. unter Bildung des Ensembles 4 an.

Mit unterbrochenen Linien sind weiters die Bereiche gezeigt, welche im und am Spül- und Füllkopf 5 und am Ventil 1 des Anschlussstutzens 31 im noch nicht zusammengeschlossenen Ensemble 4 der beiden Komponenten des Ensembles durch Einflüsse von außen kontaminiert sein können.

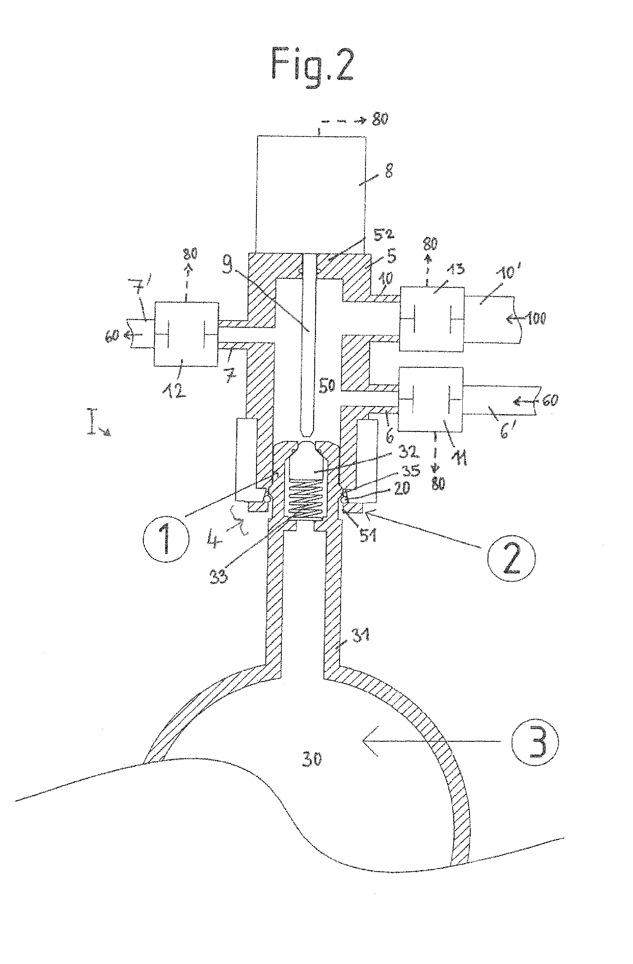

Die Fig. 2 zeigt - bei sonst gleichbleibenden Bezugszeichenbedeutungen - wie der Spül- und Füllkopf 5 unter Bildung der neuen Befüll- uns Spüleinrichtung I zu dem Ensemble 4 mit dem Anschlussstutzen 31 mit Einlassventil 1 des Behälters 3 zusammengeschlossen ist, bevor die dekontaminierende Befüllung und Spülung des Innenraums des Ensembles 4 mit überhitztem bzw. Heiß-Dampf 60 erfolgt.

Hierbei sind die Ventile 11, 12 und 13 noch geschlossen und der Einlassventil 1-Betätigungsstab 9 befindet sich in hochgefahrener Ruhestellung.

Die in Fig. 1 mit strichlierten Linienzügen gekennzeichneten Bereiche bzw. Flächen sind nach dem erfolgtem Zusammenkoppeln des Spül- und Füllkopfes 5 mit dem Anschlussstutzen 31 des Behälters 3 od. dgl. klarerweise immer noch kontaminiert.

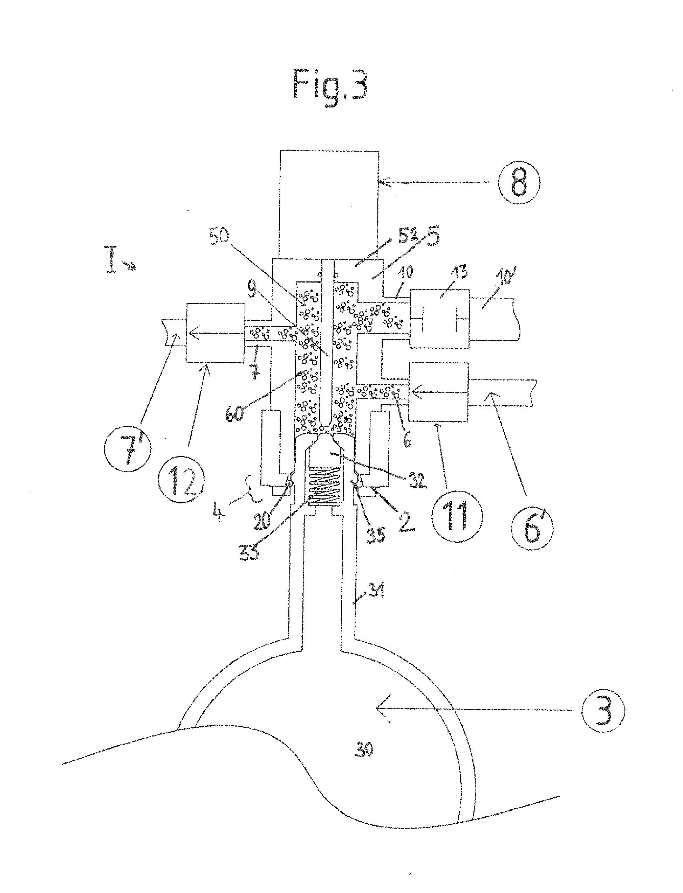

Die Fig. 3 zeigt - bei sonst gleichbleibenden Bezugszeichenbedeutungen - wie über das nun geöffnete Ventil 11 bzw. die Zuleitung 6 des Spül- und Füllkopfes 5 nun begonnen wird, Heißdampf 60 in den Innenraum 50 desselben einzulassen. Ob dieses Ventil 11 örtlich direkt am Stutzen 6 sitzt, oder an einer anderen geeigneten Stelle des Gesamtaufbaues ist je nach Anwendungsfall individuell umzusetzen. Dasselbe gilt für den Dampfableitungsstutzen 7 bzw. das dortige Sperrventil 12, Die Ventile 11 und 12 sorgen dafür, dass der Bereich der möglichen Kontamination mit dem Heißdampf 60 voll und intensiv über- und durchströmt werden kann, und nicht nur unter Heißdampf 60 gesetzt wird.

Vorzugsweise weist der Stutzen 7 und die Abführleitung 7' einen etwas kleineren Querschnitt auf als der Zuleitungsstutzen 6. So kann sichergestellt werden, dass der Heißdampf 60 den gesamten Bereich der möglichen Kontamination voll erreicht und über- und durchströmt.

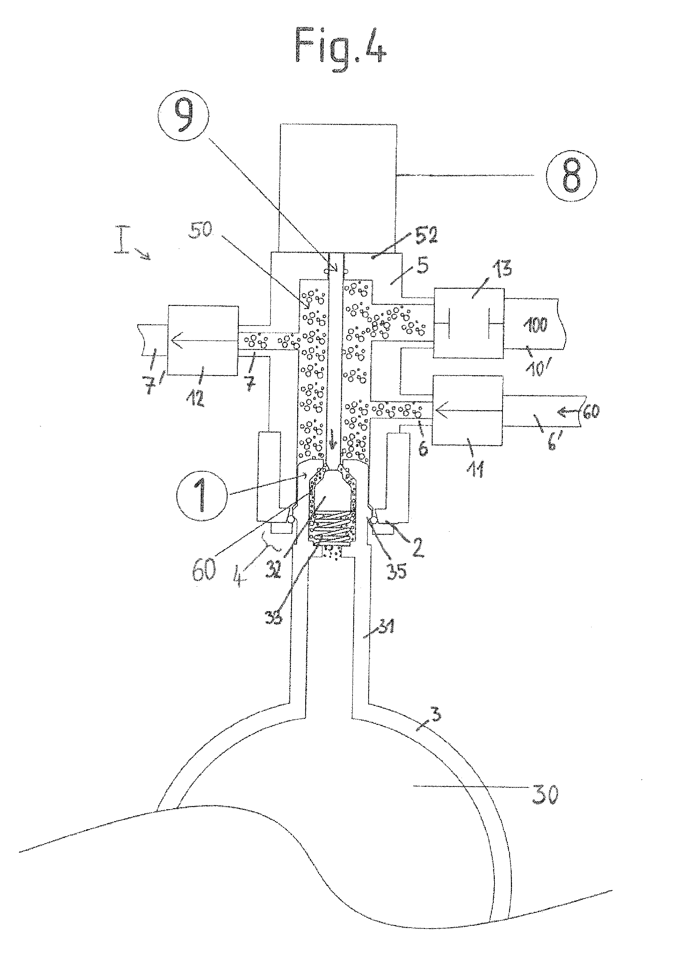

Aus Fig. 4 ist - bei sonst gleichbleibenden Bezugszeichenbedeutungen - ersichtlich, dass die Mechanik der Betätigungseinheit 8, z.B. ein Pneumatik-Zylinder, dann (mittels) des Einlassventill-Beätigungsstiftes 9, das Einlassventil 1 des Anschlussstutzens 31 des Behälters 3 öffnet, dies nachdem der gesamte kontaminationsgefährdete Bereich mittels Heißdampf 60 steril gebracht wurde. Theoretisch könnte nun mit der Förderung des fluiden Mediums 100 durch den Stutzen 10 des Spül- und Füllkopfes 5 begonnen werden. Es wird aber weiterhin Heißdampf 60 zugeführt, sodass auch der Bereich des Einlassventils 1, in welchem möglicherweise auch Kontamination erfolgt sein könnte und welcher zuvor vom Heißdampf 60 nicht erreicht werden konnte mit hoher Sicherheit voll sterilisiert wird.

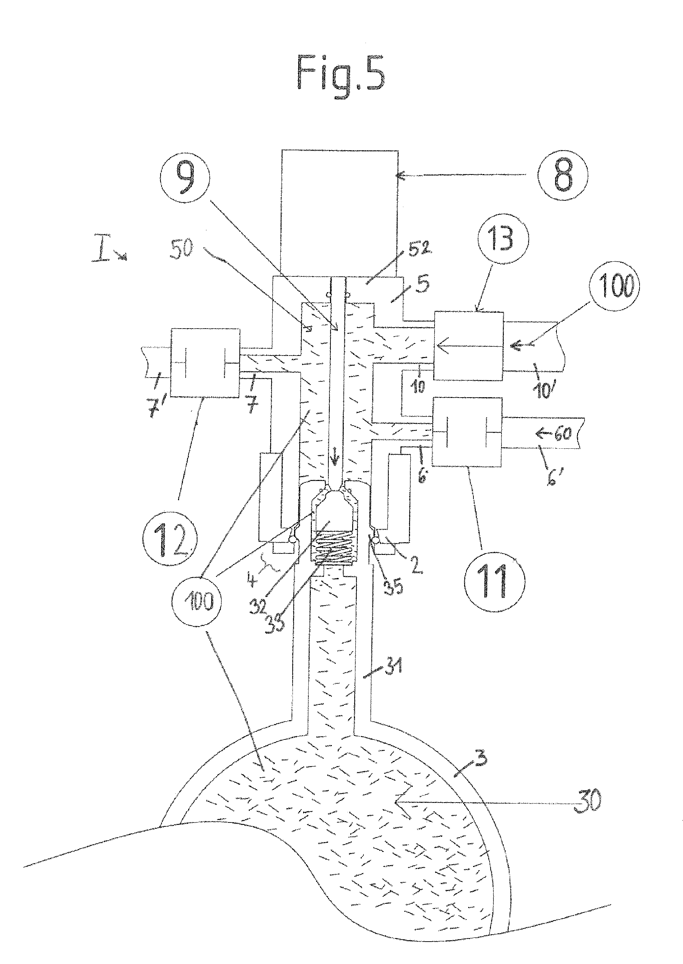

In Fig. 5 ist - bei sonst gleichbleibenden Bezugszeichenbedeutungen - dargestellt, dass alle möglicherweise kontaminierten Bereiche von Spül- und Füllkopf 5 und Innerem des Anschlussstutzens 31 mit Einlassventil 1 vollständig mittels Heißdampf 60 steril gebracht wurden.

Nun kann mit der Förderung des jeweils vorgesehenen fluiden Mediums 100 über Stutzen 10 begonnen werden. Hierbei sind die Ventile 11 u. 14 geschlossen und das Ventil 13 ist geöffnet. Das fluide Medium 100 kann nun über die voll sterilisierten Bereiche von Spül- und Füllkopf 5 und Anschlussstutzen 31 des Behälters 3 mit dem Einlassventil 1 gefördert werden, z.B. wie hier gezeigt, in den schematisch dargestellten Behälter 3.

Nachdem die Förderung des Mediums 100 in den Behälter 3 abgeschlossen ist, wird das Einlassventil 1 wieder geschlossen, indem die Mechanik der Betätigungseinrichtung 8 den Einlassventill-Betätigungsstab 9 zurückzieht und das Einlassventil 1 somit letztlich wieder geschlossen ist. Danach können Spül- und Füllkopf 5 und Anschlussstutzen 31 des Behälters 3 wieder entkoppelt werden.

Der gesamte Prozess kann, wie oben kurz beschrieben, komplett automatisch, im Halbautomatik-Betrieb oder aber händisch erfolgen. Je nach zu förderndem fluidem Medium 100 können gegebenenfalls einzelne Leitungen und Ventile entfallen. Speziell bei einem gasförmigen Medium 100 könnten die Stutzen 6 und 10 zusammengelegt werden, oder auch der Stutzen 7 komplett entfallen, weil ja nur der Heißdampf aus demselben bzw. Leitung 7' entweicht.

Dies kann aber je nach Einsatzzweck bzw. Einsatzort individuell ausgeführt werden.

Der Grundaufbau bzw. die Funktion der erfindungsgemäßen Einrichtung 100 bleibt jedoch, wie beschrieben, derselbe.

Ergänzend wird noch Folgendes ausgeführt: Die neue Einrichtung I wurde bereits in die Praxis umgesetzt und die Funktion der erfolgreichen Sterilisation wurde anhand von eingehenden mikrobiologischen Testreihen geprüft und hierbei wurde die zu 100% erfolgte Dekontamination voll bestätigt.

Der Zweck der neuen Einrichtung besteht insbesondere bei deren Einsatz in einem Lebensmittelunternehmen auch darin, die sterile Druckkontrolle bzw. -beaufschlagung und -ablassung bei der Befüllung bzw. Entleerung von sterilen bzw. steril zu haltenden Druckbehältern mit Frucht- und Gemüseprodukten vorzunehmen. Des weiteren ist auch eine sterile Druckkontrolle, die bisher mittels Manometer, welches nach Desinfektion des Spülkopfes mit Ethanol auf dem Behälter, Container od. dgl. angebracht wird, durchgeführt wurde, möglich.

Das Ziel der Erfindung besteht darin, das Risiko einer mikrobiologischen Kontamination zu minimieren oder besser: völlig auszuschalten und einen komplett sterilen Prozess beim Ankoppeln des Spül- und Füllkopfes 5 an den Anschlussstutzen 31 eines Produkt-Behälters 3 od. dgl. zu garantieren. Dieses Ziel konnte mit der beschriebenen erfindungsgemäßen Einrichtung voll erreicht werden.

## Patentansprüche

1. Steril-Befüll- bzw. Spüleinrichtung für ein kontaminationsfreies Befüllen bzw. Spülen von mit sensiblen fließfähigen Produkten, insbesondere des Lebens- und Arzneimittelsektors, zu beschickenden Behältern, Tanks, Containern, Rohren oder Rohrsystemen mit einem fluiden Medium aus der Gruppe Flüssigkeiten und Gase, insbesondere mit einem Inert- bzw. Sterilgas, wie Stickstoff oder Kohlendioxid, umfassend einen Spül- und Füllkopf für die Führung bzw. Zuführung des fluiden Mediums und einem mit dem Spül- und Füllkopf fluiddicht zu einem Ensemble verbindbaren Anschlussstutzen des Behälters od.dgl. mit Einlassventil mit nach außen hin druckfederbelastetem Ventilkörper **dadurch gekennzeichnet,**
- **dass** der, vorzugsweise im Wesentlichen zylindrische, Spül- und Füllkopf (5) zumindest drei dort seitlich einmündende mit seinem Innenraum (50) in Verbindung stehende, selbst jeweils ein Sperrventil (11, 12, 13) aufweisende, außen an flexible Rohre oder Schläuche (6', 7', 10') angeschlossene, Stutzen (6, 7 und 10) aufweist, von welchen zwei (6, 7) jeweils für Zufuhr- und Abfuhr von überhitztem bzw. Heiß-Dampf (60) und der dritte (10) für den Ein- und Durchlauf des jeweiligen Fluidmediums (100) vorgesehen, sind, wobei gegebenenfalls der Stutzen (7) einen geringeren Querschnitt aufweist als der Stutzen (6),
- **dass** er weiters an seinem offenen unteren Ende (51) mit einem Anschlussadapterstück (2) ausgebildet ist und an seinem dem offenen unteren Ende (51) gegenüberliegenden Ende (52) eine gegebenenfalls mit einer Messeinheit, beispielsweise Manometer und/oder Thermometer, für die Ermittlung von Druck und/oder Temperatur, beispielsweise im Spül- und Füllkopf-Innenraum (50), ausgestattete Betätigungseinrichtung (8) für das Hoch- oder Niederfahren eines für das Betätigen, insbesondere Öffnen, des druckfederbelasteten Ventilkörpers (32) des Einlassventils (1) am Anschlussstutzen (31) des Behälters (3) od. dgl. vorgesehenen, mittig, insbesondere achsial, in den Innenraum (59) des Spül- und Füllkopfes (5) ragenden Einlassventil-Betätigungsstiftes bzw. -stabes (9) aufweist, und
- **dass** erst nach erfolgtem, vorzugsweise übergreifendem, Zusammenschluss zum Ensemble (4) von Spül- und Füllkopf (5) mit seinem Anschluss-Adapterstück (2) mit dem Einlassventil (1) am Anschlussstutzen (31) des Behälters (3) od. dgl. über dessen Gegenadapter (32) der gesamte Innenraum (50) des Spül- und Füllkopfes (5) und der Außenraum um den Gegenadapter (32) sowie das Einlassventil (1) des Anschlussstutzens (31) des Behälters (3) od. dgl. und nach Niederfahren des Ventilbetätigungsstiftes (9) auch der Innenraum (33) des Einlassventils (1) mit dem Einlassventilkörper (32) zeitvorgegeben, insbesondere zeitablaufgesteuert, mit dem Heißdampf (60) bis zu einer voll gesicherten Sterilisierung des Inneren dieses Ensembles (4) beschickbar ist,
- wonach erst die Befüllung und Spülung des Behälters (3) od. dgl. mit dem fluiden Medium (100) vornehmbar ist.

2. Steril-Befüll- und Spüleinrichtung (I) **dadurch** h **gekennzeichnet,** dass sie eine zentrale Steuerungseinheit (80) aufweist, welche über (Funk-)Datenleitungen mit den zeit-vorgegeben, insbesondere -gesteuert, aufeinander abgestimmt betätigbaren Ventilen (11, 12) für die Heißdampf (60)-Führung und mit dem Ventil (10) für den Ein- und Durchlauf des fluiden Mediums (100) sowie mit der Betätigungseinrichtung (8) für den Einlassventil-Betätigungsstift bzw. -stab (9) datentransfer-verbunden ist.

3. Steril-Befüll- und Spülenrichtung (I) nach Anspruch 1 oder 2, **dadurch** h **gekennzeichnet,** dass an zumindest einer, insbesondere durch Kondensation des Heißdampfes (60) gefährdeten, sensiblen Stelle des Innenraums (50) des Spül- und Füllkopfes (5) ein Thermosensor angeordnet ist, der über eine Temperaturmesseinheit der Betätigungseinrichtung (8) mit der zentralen Steuerungseinheit (80) datentransfer-verbunden ist.

4. Spül- und Füllkopf (5) der Steril-Befüll- und Spülenrichtung (I) nach einem der Ansprüche 1 bis 3 für ein kontaminationsfreies Befüllen bzw. Spülen von mit sensiblen fließfähigen Produkten zu beschickenden Behältern, Tanks, Containern, Rohren oder Rohrsystemen, **dadurch** h **gekennzeichnet,** dass er im Wesentlichen Zylinderform mit zylindrischem Innenraum (50) aufweist, in welchen drei an flexible Rohre oder Schläuche angeschlossene Stutzen (6, 7 und 10) seitlich einmünden, von welchen zwei (6, 7) jeweils für Zufuhr- und Abfuhr von überhitztem bzw. Heiß-Dampf (60) und der dritte (10) für den Ein- und Durchlauf des jeweiligen Fluidmediums (100) vorgesehen, sind,
- dass er weiters an seinem offenen Ende (51) mit einem Anschlussadapterstück (2) ausgebildet ist und an seinem dem offenen Ende (51) gegenüberliegenden Ende (52) gegebenenfalls mit Druck- und/oder Temperatur-Messeinheit ausgestattete, Betätigungseinrichtung (8) für das Hoch- oder Niederfahren des für das Betätigen, insbesondere Öffnen, des druckfederbelasteten Ventilkörpers (32) des Einlassventils (1) am bzw. im Anschlussstutzen (31) des Behälters (3) od. dgl. vorgesehenen, mittig, insbesondere achsial, in den Innenraum (59) des Spül- und Füllkopfes (5) ragenden Einlassventil-Betätigungsstiftes bzw. -stabes (9) aufweist, und
- dass er (5) mit seinem Anschlussadapterstück (2) an den Anschlussstutzen (31) mit Einlassventil (1) des zu befüllenden Behälters (3) od. dgl. fluiddicht anschließbar ist.

5. Verfahren zum Steril-Befüllen und -Spülen von mit sensiblen fließfähigen Produkten, insbesondere des Lebens- und Arzneimittelsektors, beschickbaren Behältern, Tanks, Containern, Rohren oder Rohrsystemen mit einem fluiden Medium aus der Gruppe Flüssigkeiten und Gase, insbesondere mit einem Inertgas, wie Stickstoff oder Kohlendioxid, **dadurch gekennzeichnet,**
- **dass** der Spül- und Füllkopf (5) der Steril-Befüll- und Spüleinrichtung (I) gemäß einem der Ansprüche 1 bis 4 fluiddicht an den Anschlussstutzen (31) des Behälters (3) bzw. an dessen Ventil (1) mit druckfederbelastetem (34) Ventilkörper (32) angeschlossen wird,
- **dass** danach bei geschlossenem Ventil (13) des Stutzens (10) und bei geöffnetem Ventil (11) des Stutzens (6) und bei zumindest zeitweise geöffnetem Ventil (12) des Stutzens (7) überhitzter bzw. Heiß-Dampf (60) so lange in und durch den Innenraum (50) des Spül- und Füllkopfes (5) und auf die Außenflächen des Anschlussstutzens (31) des Behälters (3) von dessen geschlossenem Ventil (1) und dessen mit druckfederbelastetem (34) Ventilkörper (32) spülend geleitet wird, bis dort volle Sterilität gewährleistet ist,
- **dass** dann nach Öffnung des Einlassventils (1) des Anschlussstutzens (31) des Behälters (3) der Einlassventil-Innenraum (34) mit dem Heißdampf (60) bis zur Einreichung voller Sterilität dortselbst beaufschlagt wird, und
- **dass** schließlich nach Schließen der Ventile (11 und 12) in den Stutzen (6, 7) und nach Öffnen des Zufuhrventils (13) das fluide Medium (100) über das mittels des Ventilbetätigungsstiftes bzw. -stabes (9) geöffnet gehaltene Einlassventil (1) des Anschlussstutzens (31) des Behälters (3) od. dgl. in denselben einlaufen gelassen wird.

6. Verwendung einer Steril-Befüll- bzw. Spüleinrichtung bzw. eines Spül- und Füllkopfes gemäß einem der Ansprüche 1 bis 3 bzw. 4 für ein kontaminationsfreies Befüllen bzw. Spülen von mit sensiblen fließfähigen Produkten, insbesondere des Lebens- und Arzneimittelsektors, zu beschickenden Behältern, Tanks, Containern, Rohren oder Rohrsystemen, insbesondere unter Durchführung des Verfahrens gemäß Anspruch 5.
